(19) Europäisches Patentamt
European Patent Office
Office européen des brevets

(11) EP 4 714 651 A1

(12) **EUROPEAN PATENT APPLICATION**

(43) Date of publication:
**25.03.2026 Bulletin 2026/13**

(21) Application number: **24202533.6**

(22) Date of filing: **25.09.2024**

(51) International Patent Classification (IPC):
**B32B 27/08** (2006.01)   **A61F 13/49** (2006.01)
**A61F 13/496** (2006.01)   **A61F 13/551** (2006.01)
**B32B 27/32** (2006.01)

(52) Cooperative Patent Classification (CPC):
**B32B 27/08; A61F 13/55115; B32B 27/32;**
B32B 2250/03; B32B 2272/00; B32B 2439/46

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **23.09.2024 EP 24202075**

(71) Applicants:
• **Ontex BV**
  **9255 Buggenhout (BE)**

• **Ontex Group NV**
  **9320 Erembodegem (BE)**

(72) Inventors:
• **COBBAERT, Dries**
  **1770 Liedekerke (BE)**
• **JANSEN, Bart**
  **2640 Mortsel (BE)**

(74) Representative: **Macchetta, Andrea**
  **Ontex BV**
  **Korte Keppestraat 21**
  **9320 Erembodegem-Aalst (BE)**

(54) **POST-CONSUMER RECYCLED MATERIAL IN FLEXIBLE PACKAGES COMPRISING ABSORBENT ARTICLES**

(57)    A package comprising a bag and a stack of absorbent articles, wherein the bag comprises a multilayer plastic film, in particular multilayer plastic composite film, based on plastic recyclate (recycled plastic). The plastic film has a plastic recycled content of at least 50 wt.% based on the plastic film. The one or more absorbent articles comprise at least 5 wt.% of recycled content based on the total weight of the absorbent article excluding the absorbent core material. The package has an eco-factor of at least 0,05 according to the method herein.

FIG. 1

EP 4 714 651 A1

## Description

### TECHNICAL FIELD

The present disclosure relates to the use of post-consumer recycled (PCR) material in flexible packages, in particular packaging films. The present disclosure furthermore relates to absorbent articles comprising recycled content.

### BACKGROUND

Providing various different products in flexible packages made of polymeric film material is widely known. For example, absorbent articles, such as diapers, bibs, wipes, sanitary napkins, tampons, etc. are commonly packaged in flexible packages made of film.

In view of increasing focus on environmental aspects, sustainability of consumer products is growing, including the way in which these products are packed. Packaging material based on petrol-based raw materials, such as polymeric materials, is more and more seen as problematic by consumers and manufacturers. It is therefore not only desirable for economic, ecological and health reasons in particular, but rather also necessary to use plastic waste for the new production of plastic-based products, wherein in this context - in order to enable a sustainable economic cycle - the final properties of the products produced using plastic waste should at least substantially not deteriorate in comparison with corresponding products produced from primary plastics.

To address these concerns, different approaches have been evaluated and continue to be explored. For example, all or at least a certain proportion of the petrol-based material can be replaced with materials derived from renewable resources, such as starch. Another approach is the recycling of packaging material back into the packaging manufacturing process.

The aim of such a recycling process for plastic waste is to obtain plastic waste material which is as pure as possible (i.e. pure as plastic), since only such a plastic waste material can be processed into a high-quality plastic recyclate for further processing into plastic products. For this purpose, the plastic wastes are usually melted or sintered, in particular in the context of extrusion, injection moulding, injection moulding, sintering, intrusion processes, etc., which is why only compatible plastics of the same type can be processed in this way.

However, adding recycled materials back into the manufacturing process often negatively impacts the quality of the resulting new flexible film packaging. These adverse effects can relate to a number of properties, such as mechanical aspects (e.g. tensile strength or elongation at break), optical properties of the film material, or reduced processability, such as printing or sealing. Due to these problems, flexible film packages often comprise only relatively small amounts of recycled materials.

Consequently, there is still no possibility in the prior art of recycling or recycling plastic waste in such a way that high-performance plastic-based new materials can be provided or produced which consist at least decisively or essentially exclusively of the relevant plastic recyclates obtained from the plastic waste. Furthermore, the prior art also lacks the need to provide such new plastics which consist decisively or essentially of plastic waste which can be recycled again, so that a long-term and lasting economic cycle is formed.

It would thus be desirable to provide a recycling approach for making flexible packages which reduces or even offsets the above-mentioned negative effects while at the same time enabling use of relatively high amounts of recycled materials.

### SUMMARY

The present disclosure provides a package comprising a bag and a stack of absorbent articles, wherein the bag (10) comprises a multilayer plastic film, in particular multilayer plastic composite film, based on plastic recyclate, and wherein the plastic film has a plastic recycled content of at least 50 wt.% based on the plastic film. The one or more absorbent articles in said package comprise at least 5 wt.% of recycled content based on the total weight of the absorbent article excluding the absorbent core material. The package comprises an eco-factor of at least 0,05 according to the method herein.

### BRIEF DESCRIPTION OF THE FIGURES

FIG. 1 is a perspective view of a bag of one embodiment of the present invention.

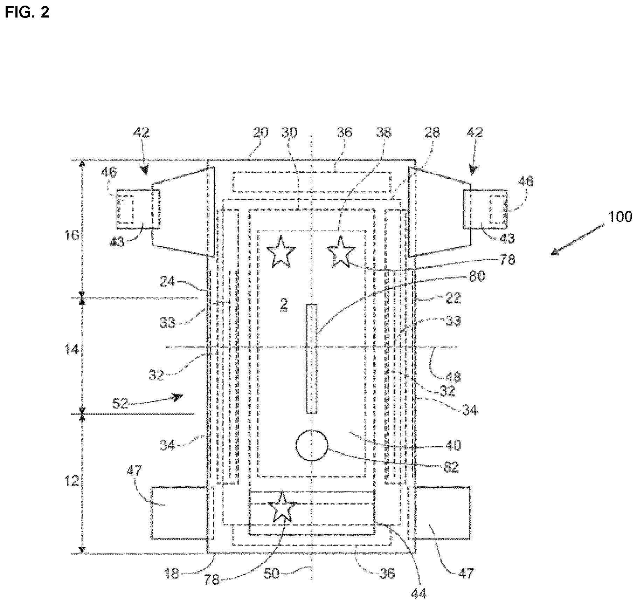

FIG. 2 is a plan view of an example absorbent article in the form of a taped diaper, garment-facing surface facing the viewer, in a flat laid-out state;

## DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

The expression "% by weight" (weight percent) otherwise referred to as "wt%", here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

The term "absorbent article" refers to a device that absorbs and contains liquid, and more specifically, refers to a device that is placed against or in proximity to the body of the wearer to absorb and contain the various wastes/exudates discharged from the body. Absorbent articles include but are not limited to diapers, adult incontinence briefs, training pants and diaper holders and liners.

The term "bio-based" may refer to a component of an absorbent article that can be produced or is derived from a renewable resource, as defined herein.

The term "bio-based content" refers to the amount of carbon from a renewable resource in a material as a percent of the mass of the total organic carbon in the material as determined by ASTM D6866-10, method B. In order to apply the methodology of ASTM D6866-10, method B, to determine the bio-based content of any absorbent article or component thereof, a sample may be ground into particulates less than about 20 mesh using known grinding methods (e.g., Wiley® mill (Thomas Scientific, LLC)), and a representative sample of suitable mass taken from the randomly mixed particles. Alternatively, if the sample is merely a layer of material, then the layer itself may be analyzed without the need for a pregrinding step. Determining the bio-based content of a component of an absorbent would first require the component to be dissected, isolated, or cleanly removed from a completed absorbent article as the first step of the analysis. The bio-based content of a component material as described herein would be the mean value of representative sampling of the component as describe above from five different absorbent articles from a given package of absorbent articles or five absorbent articles from randomly purchased packages of a given absorbent article. Note that any carbon from inorganic sources, such as calcium carbonate, is not included in determining the bio-based content of the material. See additional information below for measuring bio-sourced content in polymers. Components of the absorbent articles described in this application may be at least partially comprised of bio-sourced content as described in the following published U.S. patent applications: U.S. Pat. Appl. Pub. Nos. 2007/0219521, 2011/0139658, 2011/0139657, 2011/0152812, 2011/0139662, and 2011/0139659.

The term "disposable" may be used herein to describe absorbent articles and other products which are not intended to be laundered or otherwise restored or reused as an absorbent article or product (i.e., they are intended to be discarded after use and, preferably, to be recycled, composted or otherwise disposed of in an environmentally compatible manner).

The terms "elastic," "elastomer," and "elastomeric" may be used herein to refer to any material that is able to extend to a strain of at least 50% without breaking or rupturing when subjected to a tensile force and is able to recover substantially to its original dimensions after the force has been removed.

The term "film" refers to a sheet-like material wherein the length and width of the material far exceed the thickness of the material. Typically, films have a thickness of about 0.5 mm or less.

The term "Natural fibers" refers to elongated substances produced by plants and animals and includes animal-based fibers and plant-based fibers, as those categories are described herein. Natural fibers, as that term is used herein, include fibers harvested without any postharvest treatment step, as well as those having a post-treatment step, such as, for example, washing, scouring, bleaching, etc.

The term "Naturally-derived" materials include materials that are partially chemically altered without petroleum components and that have been minimally processed such that they not be altered to such an extent that they are substantially less biodegradable or more toxic.

The term "Plant-based fibers," as that term is used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut.

The term "renewable resource" may be used herein to refer to a natural resource that may be produced by a natural process at a rate comparable to its rate of consumption (e.g., within a 100 year time frame). The resource may be replenished naturally, or via engineered agricultural techniques. Non-limiting examples of renewable resources may include plants, animals, fish, bacteria, fungi, and forestry products. These resources may be naturally occurring, hybrids, or genetically engineered organisms. Natural resources such as crude oil, coal, and peat that take longer than 100 years to form are generally not considered to be renewable resources.

The term "recycling" or "Recycling" as used herein refers to, in particular, those processes in which raw materials are obtained from wastes which, in turn, are returned to the economic cycle, in particular are processed to give new products; these processes are also referred to as material utilization. As a result of such processes, the amounts of waste which are produced, in particular the amounts of waste which are otherwise to be incinerated or dumped, are significantly reduced, while at the same time the finite raw material supplies of the earth are protected. In this context, it should be noted that the term "Recycling" refers to the (recovery)recovery of neurogens by actual waste, but not, for example, the reuse or further use of production residues. In particular, the plastic waste to be recycled can be, for example, films or packages which, for example, were already in third-party use and have been supplied to the end customer.

The term "recyclable" may be used herein to refer to a material (e.g., plastic, paper, etc.) that has the ability to enter into established recycling streams or be used in a recycling stream avoiding the normal fate of a disposable absorbent product, namely deposition in a landfill.

The term "recyclates" as used herein refers to secondary substances as a basis for the production of (quality)new products, i.e. recycled plastics from plastic-based wastes (plastics wastes), in particular from so-called post-consumer wastes.

The term "post-consumer recycling (PCR)" as used herein refers to the recycling of so-called post-consumer wastes, in particular plastic wastes, which are produced by consumers and in particular households, offices and the stationary trade.

The term "grade purity" as used herein refers to identical plastics or identical grades of plastics with the same identification (i.e. identification in accordance with DIN EN ISO 11469 and/or in accordance with VDA standard 260) .

The term "machine direction" or "MD" may be used herein to refer to the direction of material flow through a manufacturing process. In addition, relative placement and movement of material can be described as flowing in the machine direction through a process from upstream in the process to downstream in the process. The term "cross direction" or "CD" may be used herein to refer to a direction that is generally perpendicular to the machine direction.

GENERAL DESCRIPTION OF AN ABSORBENT ARTICLE

The absorbent article (100) may comprise a front waist region (12), a crotch region (14), and a back waist region (16). The crotch region (14) may extend intermediate the front waist region (12) and the back waist region (16). The front waist region (12), the crotch region (14), and the back waist region (16) may each be ⅓ of the length of the absorbent article (100). The absorbent article (100) may comprise a front end edge (18), a back end edge (20) opposite to the front end edge (18), and longitudinally extending, transversely opposed side edges (22 and 24) and defined by the chassis (52).

The absorbent article (100) may comprise a liquid permeable topsheet (26), a liquid impermeable backsheet (28), and an absorbent core (30) positioned at least partially intermediate the topsheet (26) and the backsheet (28). The absorbent article (100) may also comprise one or more pairs of barrier leg cuffs (32) with or without elastics (33), one or more pairs of outer leg elastics (34), one or more elastic waistbands (36), and/or one or more acquisition materials (38). The acquisition material or materials (38) may be positioned intermediate the topsheet (26) and the absorbent core (30). An outer cover material (40), such as a nonwoven material, may cover a garment-facing side of the backsheet (28). The absorbent article may comprise back ears (42) in the back waist region (16). The back ears (42) may comprise fasteners (46) and may extend from the back waist region (16) of the absorbent article (100) and attach (using the fasteners (46)) to the landing zone area or landing zone material (44) on a garment-facing portion of the front waist region (12) of the absorbent article (100). The absorbent article (100) may also have front ears (47) in the front waist region (12). The absorbent article (100) may have a central lateral (or transverse) axis (48) and a central longitudinal axis (50). The central lateral axis (48) extends perpendicular to the central longitudinal axis (50).

In other instances, the absorbent article (100) may be in the form of a pant having permanent or refastenable side seams. Suitable refastenable seams are disclosed in U.S. Pat. Appl. Pub. No. 2014/0005020 and U.S. Pat. No. 9,421,137. The absorbent article may have a front waist region, a crotch region, and a back waist region. Each of the regions may be ⅓ of the length of the absorbent article (100). The absorbent article (100) may have a chassis (sometimes referred to as a central chassis or central panel) (52) comprising a topsheet (26), a backsheet (28), and an absorbent core (30) disposed at least partially intermediate the topsheet (26) and the backsheet (28), and an optional acquisition material (38). The absorbent article (100) may comprise a front belt in the front waist region (12) and a back belt in the back waist region (16). The chassis (52) may be joined to a wearer-facing surface of the front and back belts or to a garment-facing surface of the belts via one or more chassis glues or adhesives and/or one or more non-adhesive methods as described herein. Side edges of the front belt may be joined to side edges and, respectively, of the back belt to form two side seams. The side seams may be any suitable seams known to those of skill in the art, such as butt seams or overlap seams, for example. When the side seams are permanently formed or refastenably closed, the absorbent article in the form of a pant has two leg openings and a waist opening circumference. The side seams may be permanently joined using adhesives or bonds, for example, or may be refastenably closed using hook and loop fasteners, for example.

**Belts**

The front and back belts may comprise front and back inner belt layers and front and back outer belt layers having an elastomeric material (e.g., strands or a film (which may be apertured)) disposed at least partially therebetween. The elastic elements or the film may be relaxed (including being cut) to reduce elastic strain over the absorbent core or, may alternatively, run continuously across the absorbent core. The elastics elements may have uniform or variable spacing therebetween in any portion of the belts. The elastic elements may also be pre-strained the same amount or different amounts. The front and/or back belts may have one or more elastic element free zones where the chassis overlaps the belts. In other instances, at least some of the elastic elements may extend continuously across the chassis.

The front and back inner belt layers and the front and back outer belt layers may be joined using adhesives, heat bonds, pressure bonds or thermoplastic bonds. Various suitable belt layer configurations can be found in U.S. Pat. Appl. Pub. No. 2013/0211363.

The front outer belt layer and the back outer belt layer may be separated from each other, such that the layers are discrete or, alternatively, these layers may be continuous, such that a layer runs continuously from the front belt end edge to the back belt end edge. This may also be true for the front and back inner belt layers-that is, they may also be longitudinally discrete or continuous. Further, the front and back outer belt layers may be longitudinally continuous while the front and back inner belt layers are longitudinally discrete, such that a gap is formed between them.

Alternatively, instead of attaching belts to the chassis to form a pant, discrete side panels may be attached to side edges of the chassis.

**Top Sheet**

The topsheet (26) is the outermost layer or component of the absorbent article (100) that is in contact with the wearer's skin. The topsheet (26) may be joined to portions of the backsheet (28), the absorbent core (30), the barrier leg cuffs (32), and/or any other layers as is known to those of ordinary skill in the art. The topsheet (26) may be compliant, soft-feeling, and non-irritating to the wearer's skin. Further, at least a portion of, or all of, the topsheet (26) may be liquid permeable, permitting liquid bodily exudates to readily penetrate through its thickness. A suitable topsheet (26) may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., a polyester or a polyolefin, such as polyethylene (PE), polypropylene (PP), or bicomponent PE/PP fibers or mixtures thereof), or a combination of natural and synthetic fibers.

In one form of the present disclosure, the topsheet (26) may be a nonwoven material having one or multiple formed layers, nonwovens, or components comprising plant-based fibers other than wood pulp. The nonwoven may be made through well-known techniques; for example, the nonwoven may be a spunbond, a carded and air-through, hydroentangled, or calendar bonded nonwoven. The plant-based fibers may also be spun fibers made at least in part from bio-based materials. For example, the plant-based fibers may be spun poly lactic acid (PLA) fibers or bio-based polyolefin spun fibers. The plant-based fibers may be single component fibers or multi-component fibers, in which less than all of the components are plant-based fibers. One example is a bi-component fiber comprising a polyester core component and a bio-based polyethylene sheath component.

The topsheet (26) may be hydrophilic or hydrophobic or may have hydrophilic and/or hydrophobic portions or layers. If the topsheet (26) is hydrophobic, typically apertures will be present so that bodily exudates may pass through the topsheet. The topsheet (26) may have a basis weight of from about 10 gsm to about 40 gsm.

In a embodiment, the topsheet (26) may comprise a bio-based polyolefin with a bio-based content from about 5% to about 100% or from about 10% to about 100% or from about 25% to about 100% or from about 40% to about 100% or from about 50% to about 100% or from about 75% to about 100% or from about 90% to about 100%, using ASTM D6866-10, method B.

Backsheet

The backsheet (28) is generally that portion of the absorbent article (100) positioned proximate to the garment-facing surface of the absorbent core (30). The backsheet (28) may be joined to portions of the topsheet (26), the outer cover material (40), the absorbent core (30), and/or any other layers of the absorbent article (100) by any attachment methods known to those of skill in the art. The backsheet (28) prevents, or at least inhibits, the bodily exudates absorbed and contained in the absorbent core (30) from soiling articles such as bedsheets, undergarments, and/or clothing. The backsheet (28) is typically liquid impermeable, or at least substantially liquid impermeable. The backsheet (28) may, for example, be or comprise a thin plastic backsheet film, such as a thermoplastic film having a thickness of about 0.012 mm to about 0.051 mm. Other suitable backsheet (28) materials may include breathable materials or additives as described herein (e.g. calcium carbonate particles) inducing microporous zones in the backsheet film, which permit vapors to escape from the absorbent article, while still preventing, or at least inhibiting, bodily exudates from passing through the backsheet (28). Backsheet films may optionally comprise bio-based materials, such as, for example, bio-based polyethylene, bio-based polypropylene, or other bio-based polyolefins.

In an embodiment, the backsheet (28) may comprise a bio-based polyolefin with a bio-based content from about 5% to about 100% or from about 10% to about 100% or from about 25% to about 100% or from about 40% to about 100% or from about 50% to about 100% or from about 75% to about 100% or from about 90% to about 100%, using ASTM D6866-10, method B.

**Outer Cover Material**

The outer cover material (sometimes referred to as a backsheet nonwoven web) (40) may comprise one or more nonwoven materials joined to the backsheet (28) and that covers the backsheet (28). The outer cover material (40) is typically the layer facing outward-i.e., towards garments or undergarments, when present, and away from the wearer's skin. A caregiver interacts significantly with the outer cover material when holding/comforting the wearer and/or changing the absorbent article. The outer cover material (40) forms at least a portion of the garment-facing surface of the absorbent article (100) and effectively "covers" the backsheet (28) so that film is not present on the garment-facing surface. The outer cover material (40) may comprise a bond pattern, apertures, and/or three-dimensional features.

In one form, the outer cover material (40) may be a carded nonwoven or a multi-layered nonwoven comprising carded layers and one or more spunbond layers. The outer cover nonwoven (40) may comprise a combination of plant-based fibers and synthetic fibers that are not plant-based. For example, the nonwoven may comprise both polypropylene fibers and cotton fibers; see, for example, U.S. Pat. Appl. Pub. No. 2017/0203542. The cotton content may range from about 3%, 5%, 10%, or 15% to about 50%, by weight of the nonwoven. When synthetic fibers such as polypropylene are employed, it is preferred that the polypropylene be non-phthalate catalyst polypropylene fibers. Furthermore, the polypropylene fibers may comprise bio-based polyethylene, bio-based polypropylene, or other bio-based polyolefins where such bio-based polyolefin is also phthalate free.

In another form, the outer cover material (40) may comprise a hydroentangled, dual layer nonwoven web, in which one layer comprises a polypropylene spunbond web having a basis weight in the range of from about 5 to 15 gsm and the other layer comprises a carded nonwoven web comprising cotton fibers and a basis weight in the range of from about 10 to about 25 gsm. In a further form, the outer cover material 40 may comprise an air through carded nonwoven web comprising a blend of polypropylene fibers and cotton fibers. The cotton fibers may be included in these nonwoven webs in an amount of about greater than or less than 5%, 10%, or 15% to about 20%, 30%, or 50%, by weight of the overall nonwoven web.

**Absorbent Core**

As used herein, the term "absorbent core" (30) refers to the component of the absorbent article having the most absorbent capacity and that comprises an absorbent material. An absorbent core (30) material may be positioned within a core bag or a core wrap or cover made from, for example, a nonwoven web. The absorbent core (30) material may be profiled or not profiled, depending on the specific absorbent article (100). The absorbent core (30) may comprise, consist essentially of, or consist of, a core wrap, absorbent core (30) material, and core glue or adhesive enclosed within the core wrap. The core wrap may be constructed with one or more nonwoven webs. These nonwoven webs may comprise non-phthalate catalyst polymers. The nonwoven webs may also comprise bio-based materials, including plant-based fibers and/or bio-based materials such as bio-based polyolefins.

The absorbent core (30) material may comprise superabsorbent polymers, a mixture of superabsorbent polymers and air felt (also referred to herein as absorbent pulp), and/or only air felt. The absorbent core (30) material may comprise just the absorbent core (30) materials alone and not include the core wrap, core wrap nonwovens, core glues or adhesives enclosed within the core wrap. The core glue or adhesive may be used to immobilize the superabsorbent polymer particles within the core wrap. In some instances, the absorbent core (30) material may comprise at least 50%, at least 60%, at least 70%, at least 80%, at least 85%, at least 90%, at least 95%, at least 99%, or up to 100% superabsorbent polymers, by weight of the absorbent core (30) material. In some instances, the absorbent core (30) material may be free of air felt, or at least mostly free of air felt. The absorbent core (30) periphery, which may be the periphery of the core wrap, may define any suitable shape, such as rectangular "T," "Y," "hour-glass," or "dog-bone" shaped, for example. An absorbent core (30) periphery having a generally "dog bone" or "hour-glass" shape may taper along its width towards the crotch region (14) of the absorbent article (100).

The absorbent core (30) may have areas having little or no core absorbent material, where a wearer-facing surface of the core wrap may be joined to a garment-facing surface of the core wrap. These areas having little or no core absorbent material may be referred to as "channels". These channels can embody any suitable shapes and any suitable number of channels may be provided. In other instances, the absorbent core (30) may be embossed to create the impression of channels.

The superabsorbent polymers of the absorbent core (30) material may comprise a bio-based acrylic acid. In some examples, the superabsorbent polymers may have a bio-based content of from about 5% to about 100%, from about 10% to about 100%, from about 25% to about 100%, from about 40% to about 100%, from about 50% to about 100%, from about 75% to about 100%, or from about 90% to about 100% and all ranges subsumed therein. Superabsorbent polymers comprising a desired bio-based content may be obtained, for example, by mixing acrylic acid derived from conventional (i.e., petroleum-based) sources with bio-based acrylic acid and forming the superabsorbent polymers with the acrylic acid mixture. Superabsorbent polymers comprising the desired bio-based content may also be obtained, for example, by mixing superabsorbent polymers made from conventional acrylic acid with superabsorbent polymers made from bio-based acrylic acid.

Bio-based acrylic acid may be produced using one or more bio-based intermediates. For example, production of conventional, petroleum-based acrylic acid may involve use of petroleum naphtha derived from refining of crude oil to produce propane and/or propene, which may then be oxidized to produce acrylic acid. Bio-based naphtha, which may be derived from renewable materials such plant oils (e.g., palm oil, tall oil, etc.) and animal fat, including waste oils and fats, may be used to produce propane that is then oxidized to produce bio-based acrylic acid. Bio-based propane may be produced from similar renewable sources such as plant and animal waste products and may also occur as a waste product from biofuel production. This bio-based propane may then be oxidized to produce bio-based acrylic acid. In addition, bio-based acrylic acid may be produced (directly) by dehydration of bio-based lactic acid or 3-hydroxy propionic acid, which may be derived from, for example, microbial fermentation of plant-based sources (e.g., corn, rice, waste paper, etc.) and from waste products such as glycerol obtained from biofuel production. These methods may produce acrylic acid of high purity, i.e., a purity of about 98 weight % acrylic acid or higher. While not wishing to be bound by theory, higher purity acrylic acid is critical for achieving high performance superabsorbent polymer characteristics, as well as consumer acceptable performance by the absorbent article (100). Bio-based acrylic acid and methods of production are further described in U.S. Pat. Appl. Pub. No. 2007/0219521 and U.S. Pat. Nos. 8,703,450; 9,630,901; and 9,822,197.

**Barrier Leg Cuffs/Leg Elastics**

The absorbent article (100) may comprise one or more pairs of barrier leg cuffs (32) and one or more pairs of outer leg elastics (34). The barrier leg cuffs (32) may be positioned laterally inboard of leg elastics (34). Each barrier leg cuff (32) may be formed by a piece of nonwoven web as described herein that is bonded via a chassis glue or adhesive and/or one or more non-adhesive methods as described herein to the absorbent article (100) so that the barrier leg cuff (32) extends upwards from a wearer-facing surface of the absorbent article (100) and provides improved containment of body exudates approximately at the junction of the torso and legs of the wearer. Useful materials may include, but are not limited to, spunbond-meltblown-spunbond (SMS) nonwoven webs. Such webs may comprise non-phthalate catalyst polypropylene fibers and/or plant-based fibers. Furthermore, these nonwoven webs may also comprise bio-based materials, including plant-based fibers and/or bio-based materials such as bio-based polyolefins.

The barrier leg cuffs (32) are delimited by a proximal edge joined directly or indirectly to the topsheet (26) and/or the backsheet (28) and a free terminal edge, which is intended to contact and form a seal with the wearer's skin. The barrier leg cuffs (32) may extend at least partially between the front end edge (18) and the back end edge (20) of the absorbent article (100) on opposite sides of the central longitudinal axis (50) and may be at least present in the crotch region (14). The barrier leg cuffs (32) may each comprise one or more elastics (e.g., elastic strands or strips) (33) near or at the free terminal edge. These elastics cause the barrier leg cuffs (32) to help form a seal around the legs and torso of a wearer. The outer leg elastics (34) extend at least partially between the front end edge (18) and the back end edge (20) of the absorbent article (100). The leg elastics (34) essentially cause portions of the absorbent article (100) proximate to the chassis side edges to help form a seal around the legs of the wearer. The leg elastics (34) may extend at least within the crotch region (14). The leg elastics (34) may be latex-free.

**Elastic Waistband**

The absorbent article (100) may comprise one or more elastic waistbands (36). The elastic waistbands (36) may be positioned on the garment-facing surface or the wearer-facing surface. As an example, a first elastic waistband may be present in the front waist region (12) near the front belt end edge and a second elastic waistband may be present in the back waist region (16) near the back end edge. The elastic waistbands (36) may comprise one or two layers of nonwoven webs with an elastic film or elastic strands therebetween. The elastic waistbands (36) may aid in sealing the absorbent article (100) around a waist of a wearer and at least inhibiting bodily exudates from escaping the absorbent article (100) through the waist opening circumference. In some instances, an elastic waistband (36) may fully surround the waist opening circumference of an absorbent article (100).

**Acquisition/Distribution Materials**

One or more acquisition and/or distribution materials (also referred to herein as "acquisition material(s)") (38) may be present at least partially intermediate the topsheet (26) and the absorbent core (30). The acquisition materials (38) are typically hydrophilic materials that provide significant wicking of bodily exudates. These materials may dewater the topsheet (26) and quickly move bodily exudates into the absorbent core (30). The acquisition materials (38) may comprise one or more nonwoven materials, foams, cellulosic materials, cross-linked cellulosic materials, air laid cellulosic nonwoven materials, spunlace materials, or combinations thereof, for example. The cellulosic materials and cross-linked cellulosic materials may be bleached, but preferably not via chlorine-bleaching so as to be total chlorine free. Hydrogen peroxide is one exemplary bleaching material that is useful in making acquisition materials that are totally chlorine-free. In other embodiments, acquisition and/or distribution materials may comprise non-phthalate catalyst polypropylene fibers and/or plant-based fibers. Furthermore, these nonwoven webs may also comprise bio-based materials, including plant-based fibers and/or bio-based materials such as bio-based polyethylene, bio-based polypropylene, other bio-based polyolefins, bio-based polyesters, and combinations thereof including bi-component fiber configurations.
In some instances, portions of the acquisition materials (38) may extend through portions of the topsheet (26), portions of the topsheet (26) may extend through portions of the acquisition materials (38), and/or the topsheet (26) may be nested with the acquisition materials (38). Typically, an acquisition material (38) may have a width and length that are smaller than the width and length of the topsheet (26). The acquisition material (38) may be a secondary topsheet in the feminine pad context. The acquisition material (38) may have one or more channels as described above with reference to the absorbent core (30) (including the embossed version). The channels in the acquisition material (38) may align or not align with channels in the absorbent core (30). In an example, a first acquisition material may comprise a nonwoven material and as second acquisition material may comprise a cross-linked cellulosic material.

**Landing Zone**

The absorbent article (100) may have a landing zone area (44) that is formed in a portion of the garment-facing surface of the outer cover material (40). The landing zone area (44) may be in the back waist region (16) if the absorbent article (100) fastens from front to back or may be in the front waist region (12) if the absorbent article (100) fastens back to front. In some instances, the landing zone (44) may be or may comprise one or more discrete nonwoven materials that are attached to a portion of the outer cover material (40) in the front waist region (12) or the back waist region (16) depending upon whether the absorbent article (100) fastens in the front or the back. In essence, the landing zone (44) is configured to receive the fasteners (46) and may comprise, for example, a plurality of loops configured to be engaged with, a plurality of hooks on the fasteners (46), or vice versa. Landing zone materials, landing zone nonwovens, landing zone nonwovens comprising loops, and fastener hooks may contain polymers containing bio-based content.

**Front and Back Ears**

The absorbent article (100) may have front (47) and/or back ears (42) in a taped diaper context. Only one set of ears may be required in most taped diapers. The single set of ears, e.g., the back ears, may comprise tape tabs comprising fasteners (also referred to herein as ear hooks) configured to engage the landing zone or landing zone area (44). If two sets of ears are provided, in most instances, only one set of the ears may have fasteners (46), with the other set being free of fasteners (46). The ears, or portions thereof, may be elastic or may have elastic panels. In an example, a stretch or elastic film or stretch or elastic strands may be positioned intermediate a first nonwoven web and a second nonwoven web. In one embodiment, the back set of ears (42) are made from first and second nonwoven webs with an elastic film or elastic strands therebetween, while the front set of ears (47) are made from a generally non-elastic nonwoven web. The nonwoven materials may comprise non-phthalate catalyst polypropylene fibers. Furthermore, these nonwoven materials or webs may also comprise bio-based materials, including plant-based fibers and/or bio-based materials such as bio-based polyolefins.
The elastic film may or may not be apertured. The ears may be shaped. The ears may be integral (e.g., extension of the outer cover material, the backsheet, and/or the topsheet) or may be discrete components attached to a chassis of the absorbent article on a wearer-facing surface, on the garment-facing surface, or intermediate the two surfaces.

**Adhesives**

Adhesives as described herein may be employed to affix one component to another component in the absorbent article's final assembly. Adhesives may also be employed to immobilize sub-components, such as, for example, particulate matter within an absorbent core component. The adhesives in some instances may be devoid of added fluorescence. Furthermore, adhesive may also comprise bio-based materials, including bio-based materials such as bio-based polyolefins, bio-based polyethylenes, bio-based polypropylenes, bio-based alpha olefin polyolefins, and combinations thereof.

PACKAGES

The absorbent articles (100) of the present disclosure may be placed into packages. The packages may comprise a polymeric bag (10) and an optional carton surrounding at least a portion of the polymeric bag (10). The polymeric bag (10) may comprise bio-based polyolefin in some forms. The optional carton may be made of fiberboard and may contain recycled material or a blend of recycled and virgin material. Each package may comprise a plurality of absorbent articles (100).

Communication in the form of graphics and/or indicia relating to properties of the absorbent articles (100) may be formed on, printed on, positioned on, and/or placed on outer portions of the packages. For example, the communication may relate to the absence of certain undesirable materials, such as chlorine, perfume, scent, fragrance, lotion, non-phthalate-catalyst polypropylene, adhesives having added florescence, and green number 7 dye. The communication may also relate to features of the contained absorbent articles (100), such as, that the absorbent articles (100) have cotton (via cotton seal icon) or plant-based materials.

The absorbent articles (100) may be packed under compression so as to reduce the size of the packages, while still providing an adequate amount of absorbent articles (100) per package. By packaging the absorbent articles (100) under compression, caregivers can easily handle and store the packages, while also providing distribution savings to manufacturers owing to the size of the packages.

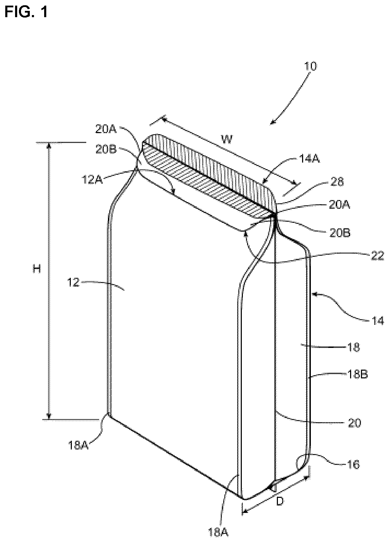

In an embodiment, as exemplified in FIG. 1, a bag (10) comprising a front surface (120) and a rear surface (140) extending upwardly from a bottom surface (160), which can be flattenable, especially when formable material is used to construct the article. Two side surfaces (180) each can be an expandable gusset (200), which can include a region of the side surface that folds to form a crease, therein connecting front surface (120) and rear surface (140) at opposite sides of bag (10). The bag (10) also can have a flattenable bottom surface (160) that can be formed by connecting front surface (120), rear surface (140), and side surfaces (180). Bag (10) also can have a top surface (220) formable by connecting front surface (120), rear surface (140), side surfaces (180), and located opposite flattenable bottom surface (160). The bag (10) has a height H, a width W, a depth D. The height H, width W, and depth D of bag (10) are defined and measured as internal dimensions of bag (10) , measured from the inside surfaces of the bag. The height H is measured along the front or rear face of the bag (10) from the inside edge of the bottom seal to the top edge of the bag (10), not including any closure. The width is measured across the front or rear face of the bag (10) from inside of edge seal to inside of edge seal at a midpoint of the height of the bag. The depth is measured across either side surface from edge seal to edge seal at a midpoint of the height of the bag. The height H, width W, and depth D define an internal volume V.

The bag (10) can be sealable by methods commonly known in the art, such as, for example, heat sealing, along at least one edge of a side surface (180a), (180b), bottom surface (160), and top surface (220). The bag (10) can also be sealed at each edge (180a), (180b). Such a bag, sealed at each of the four side edges, can be commonly referred to as a "quad seal" bag. A quad seal bag (10) is one embodiment described herein. The seals of the bag (10) can have a seal strength of at least about 1,600 pounds per square inch. The bag (10) can comprise a plastic laminate having a thickness of from about 130 to about 200 microns. The plastic laminate can have an impact dart-drop resistance of at least about 6.2 grams/mil, and a tear resistance of at least about 7.9 grams/mil and 10.8 grams/mil in the machine direction and cross direction respectively.

In a preferred embodiment, the bag (10) comprises a multilayer plastic film, in particular multilayer plastic composite film, based on plastic recyclate (recycled plastic) wherein the plastic film has a plastic recycled content of at least 50 wt. % (% by weight) based on the plastic film. The bag (10) further comprises one or more absorbent articles comprising at least 5 wt.% of recycled content (% by weight) based on the total weight of the absorbent article excluding the absorbent core material and an eco-factor of at least 0,05 according to the method described herein. Advantageously, a high recyclate content results in lower usage of fossil starting material or raw materials. Furthermore, there is a high energy efficiency since the corresponding plastics do not have to be produced from the corresponding fossil starting materials/raw materials.

In an embodiment, the eco-factor is at least 0,10; preferably at least 0,15; more preferably 0,20; even more preferably at least 0,25; even more preferably at least 0,30; even more preferably at least 0,35; even more preferably at least 0,40; even more preferably at least 0,45; even more preferably at least 0,50; even more preferably at least 0,55; even more preferably at least 0,60; even more preferably at least 0,65; even more preferably at least 0,70; even more preferably at least 0,75; most preferably between about 0,80 and 0,99.

Advantageously a higher eco-factor is the result of a greater amount of recycled content in the package. This is desirable for economic, ecological and health reasons.

In an embodiment, the plastic film comprises a layered composite of at least three film layers bonded to one another, in particular bonded and/or coextruded by means of coextrusion. Preferably, the plastic film and/or layer composite comprises a first outer film layer and a second outer film layer and a carrier film layer arranged between the first outer film layer and the second outer film layer.

In an embodiment, the multilayer plastic film has a (total) plastic content in the range from 80 wt.% to 99,9 wt.%, in particular in the range from 82,5 wt.% to 99,8 wt.%, preferably in the range from 85 wt.% to 99,8 wt.%, based on the plastic film.

In an embodiment, the plastic film has a plastic recycled content of at least 80 wt.% based on the plastic film. Advantageously, high amounts of recycled content result in a higher eco-factor.

In an embodiment, the plastic film and/or layer composite has a thickness in the range from 10 $\mu$m to 300 $\mu$m, in particular in the range from 15 $\mu$m to 200 $\mu$m, preferably in the range from 20 to 180 $\mu$m, even more preferably from 25 $\mu$m to 175 $\mu$m, even more preferably from 30 $\mu$m to 170 $\mu$m, even more preferably from 30 $\mu$m to 160 $\mu$m, even more preferably from 30 $\mu$m to 150 $\mu$m, even more preferably from 30 $\mu$m to 140 $\mu$m, even more preferably from 30 $\mu$m to 130 $\mu$m, even more preferably from 30 $\mu$m to 120 $\mu$m, even more preferably from 30 $\mu$m to 110 $\mu$m, even more preferably from 30 $\mu$m to 100 $\mu$m, even more preferably from 30 $\mu$m to 90 $\mu$m, even more preferably from 30 $\mu$m to 80 $\mu$m, even more preferably from 30 $\mu$m to 70 $\mu$m, most preferably from 30 $\mu$m to 60 $\mu$m as measured according to ISO 4593:1993.

In an embodiment, the plastic film and/or layered composite comprises at least substantially only one single plastic grade according to DIN EN ISO 11469, said plastic grade having a grade purity of at least 90 wt.%.

In an embodiment, the plastic film may be formed of polyolefin. For example, the polymeric film may be formed of polypropylene, polyethylene, or combinations thereof. A suitable polyethylene may be LDPE, LLDPE, MDPE, polyethylene based on Ziegler-Natta polymerization, and the like.

In an embodiment, the package comprises one or more absorbent articles (100) wherein the absorbent core (30) material comprises superabsorbent polymers with a bio-based content from about 5% to about 100% using ASTM D6866-10, method B. Given there may be a limited amount of petroleum in the world, it is prudent to create absorbent articles (100) comprising components derived from bio-based means or means which are not dependent upon materials for chemicals derived from petroleum sources.

In an embodiment, the package comprises one or more absorbent articles wherein at least one of the topsheet or the backsheet and at least one of the backsheet film, the backsheet nonwoven web, the landing zone, the stretch ear film, the back ear nonwoven web, the tape tabs, the ear hooks, the front ear nonwoven web, the waist feature nonwoven web, the waist feature elastics, the barrier leg cuff nonwoven web, the outer leg elastics, the acquisition material, the core cover nonwoven web, the chassis adhesives, the core adhesives, or the elastic waist panels comprise a bio-based polyolefin with: a bio-based content from 5% to 100% using ASTM D6866-10, method B; and a purity of about 98 weight % polyolefin and less than 2 weight % impurities. Given there may be a limited amount of petroleum in the world, it is prudent to create absorbent articles (100) comprising components derived from bio-based means or means which are not dependent upon materials for chemicals derived from petroleum sources.

In an embodiment, the package comprises one or more absorbent articles (100) wherein said absorbent articles (100) comprise at least 6 wt.% of recycled content, preferably at least 7 wt.%; more preferably at least 8 wt.%; even more preferably at least 9 wt.%; even more preferably at least 10 wt.%; even more preferably at least 12 wt.%; even more preferably at least 15 wt%, even more preferably at least 20 wt.%, even more preferably at least 25 wt.%, even more preferably at least 30 wt.%, even more preferably at least 35 wt.%, even more preferably at least 40 wt.%, even more preferably at least 45 wt.%, even more preferably at least 50 wt.%, even more preferably at least 55 wt.%, even more preferably at least 60 wt.%, more preferably at least 70 wt.%, even more preferably at least 80 wt.%, most preferably at least 90 wt.% of recycled content based on the total weight of the absorbent article (100) excluding the core absorbent (30) material. Advantageously, a high recycled content results in lower usage of fossil starting material or raw materials. Furthermore, there is a high energy efficiency since the corresponding plastics do not have to be produced from the corresponding fossil starting materials/raw materials.

In an embodiment, the package comprises one or more absorbent articles (100) wherein said absorbent articles (100) comprise a polyolefin selected from polyethylene and polypropylene, preferably polypropylene, and a polyolefin content of at least 90% by weight, based on a total weight of the absorbent article (100° excluding the absorbent core (30) material; and wherein recycled polyolefin recovered from an entirety of the absorbent article (100) via a recycling operation comprises a polyolefin content of at least about 90 wt.%, based on a total weight of the absorbent article (100) excluding the absorbent core (30) material. Advantageously, a high recycled content results in lower usage of fossil starting material or raw materials. Furthermore, there is a high energy efficiency since the corresponding plastics do not have to be produced from the corresponding fossil starting materials/raw materials.

**METHODS**

Eco-factor

The eco-factor is defined as the multiplication of the amount (% by weight) of recycled content of the bag (10) divided by 100% with the amount (% by weight) of recycled content of the one or more absorbent articles (100) excluding the absorbent core (30) material divided by 100%.

Eco-factor = (wt. % of recycled content of the bag (10) / 100%)/(wt. % of recycled content of the one or more absorbent articles excluding the absorbent core material/100%)

Validation of Polymers Derived from Renewable Resources

Assessment of the renewably-based carbon in a material may be performed through standard test methods. Using radiocarbon and isotope ratio mass spectrometry analysis, the bio-based content of materials may be determined. ASTM International, formally known as the American Society for Testing and Materials, has established a standard method for assessing the bio-based content of materials. The ASTM method is designated ASTM D6866-10, method B.
Assessment of the materials described herein may be done in accordance with ASTM D6866-10, method B. The mean values quoted in a report from this type of analysis encompass an absolute range of 6% (plus and minus 3% on either side of the bio-based content value) to account for variations in end-component radiocarbon signatures. It is presumed that all materials are present day or fossil in origin and that the bio-based content result is the amount of bio-based component "present" in the material. One company providing reports on or performing bio-based content analysis of a component of an absorbent article according to ASTM D6866-10, method B is Beta Analytic Inc. of Miami, Fla.

Method to evaluate the olefin content of an absorbent article

This method may be used to determine: (1) the olefin content of an individual component harvested from an absorbent article; and (2) the overall olefin content of an absorbent article comprising the individual harvested components.
The absorbent article is to be deconstructed, removing the core materials, and separating each of the other structures into distinctive individual components. The components are weighed and analyzed using the following standard methods: (1) Differential Scanning Calorimetry (DSC) (ASTM D3418.21296); and (2) FT (Fourier transformed) Infrared Spectroscopy with ATR (Attenuated total reflection) (ASTM E1252.29254).
Unless otherwise specified, all tests described herein, including those described in the detailed description, are conducted on samples that have been conditioned in a conditioned room at a temperature of 73° F. $\pm$ 2° F. (23° C. $\pm$ 1° C.) and a relative humidity of 50% ($\pm$ 2%) for 2 hours prior to the test. All tests are conducted in such conditioned room(s).

**Sample Preparation**

The absorbent article is deconstructed into the individual components, and residual adhesive on each component is removed via dissolution in any suitable solvent, for example, Tetrahydrofuran (THF)-Stabilized (HPLC Grade). The components are air-dried for 30 minutes to remove traces of solvent. The weight of each individual component is recorded in gram (g) to the nearest 0.01 g. The steps in harvesting the components are shown below:

**Procedure**

1. Obtain a 3-4 liter beaker;
2. Obtain one 4 liter bottle of solvent;
3. In a hood, transfer 1 liter of solvent into the 3-4 liter beaker;
4. Apply C-clamps to the top and bottom of the absorbent article;
5. Take one absorbent article and submerge in the 1 liter of solvent;
6. Place beaker on shaking table and stir gently for 15 minutes;
7. Let solution with the absorbent article sit for 5 additional minutes;
8. Take the absorbent article out of solvent solution;
9. Carefully squeeze solvent solution out of the absorbent article;
10. Place the absorbent article on a glass dish (topsheet side up);

11. Carefully dissemble product, take note of the order of the components;

12. Place components on racks in hood and insure correct ID of components;

13. Let components air dry in hood for a minimum of 15 minutes;

14. Once components are completely dry, proceed with dimensional analysis; and

15. Record the weight and the physical dimensions of each of the individual materials

**Sampling**

For the DSC from each component a sample is cut not exceeding 3 mm in diameter. For the ATR FT IR a sample sufficient in size to cover the IR crystal is cut.

**Analysis**

Each component is then analyzed by Differential Scanning Calorimetry (ASTM D3418.21296) and FT (Fourier transformed) Infrared Spectroscopy with ATR (Attenuated total reflection) (ASTM E1252.29254). The resulting thermograms and FTIR spectra are evaluated to determine the percentage of the component specimen consistent with an olefin. The comparison of the melting peak area of the second heat in the DSC will provide the ratio of olefin to non-olefinic material in the individual component.

**Result**

The percentage olefin content of each individual component of the absorbent article multiplied by its mass are combined and then divided by the total weight of the article minus the weight of the absorbent material to yield the overall olefin percentage of the article. The individual components olefin percentage and the overall olefin percentage is reported to the nearest 1%.

**Claims**

1. A package comprising a bag (10) and a stack of absorbent articles (100),

   - wherein the bag (10) comprises a multilayer plastic film, in particular multilayer plastic composite film, based on plastic recyclate,
   - wherein the plastic film has a plastic recycled content of at least 50 wt.% based on the plastic film,
   - wherein one or more absorbent articles (100) comprise at least 5 wt.% of recycled content based on the total weight of the absorbent article (100) excluding the absorbent core (30) material;
   and
   - an eco-factor of at least 0,05 according to the method herein.

2. A package comprising a bag (10) and a stack of absorbent articles (100) selected from group consisting of disposable diapers and pants,

   - wherein the bag (10) comprises a multilayer plastic film, in particular multilayer plastic composite film, based on plastic recyclate,
   - wherein the plastic film has a plastic recycled content of at least 80 wt.% (% by weight) based on the plastic film.

3. A package according to Claim 1 wherein the eco-factor is at least 0,10; preferably at least 0,15; more preferably at least 0,20; even more preferably at least 0,25; even more preferably at least 0,30; even more preferably at least 0,35; even more preferably at least 0,40; even more preferably at least 0,45; even more preferably at least 0,50; even more preferably at least 0,55; even more preferably at least 0,60; even more preferably at least 0,65; even more preferably at least 0,70; even more preferably at least 0,75; most preferably between about 0,80 and 0,99.

4. A package according to any of the preceding Claims wherein the absorbent articles (100) are selected from disposable diapers and pants and wherein said absorbent articles (100) comprise one or more of the following: absorbent core material (30), a topsheet (26), a backsheet (28), a backsheet film, a backsheet nonwoven, a landing zone (44), a stretch ear film, a back ear nonwoven, tape tabs (46), ear hooks, a front ear nonwoven (47), a waist feature nonwoven, waist feature elastics, barrier leg cuff nonwoven (32), outer leg elastics (34), an acquisition material (38), a core wrap nonwoven, chassis adhesives, core adhesives, elastic waist panels.

5. A package according to any of the preceding Claims wherein the plastic film comprises a layered composite of at least three film layers bonded to one another, in particular bonded and/or coextruded by means of coextrusion.

6. A package according to any of the preceding Claims wherein the plastic film and/or layer composite comprises a first outer film layer and a second outer film layer and a carrier film layer arranged between the first outer film layer and the second outer film layer.

7. A package according to any of the preceding Claims, wherein the multilayer plastic film has a (total) plastic content in the range from 80 wt% to 99,9 wt.%, in particular in the range from 82,5 wt.% to 99,8 wt.%, preferably in the range from 85 wt.% to 99,8 wt.%, based on the plastic film.

8. A package according to any of the preceding Claims wherein the plastic film has a plastic recycled content of at least 80 wt.% based on the plastic film.

9. A package according to any of the preceding Claims wherein the one or more absorbent articles (100) comprise at least 10 wt.% of recycled content; preferably at least 15 wt.%, more preferably at least 20 wt.%, even more preferably at least 25 wt.%, even more preferably at least 30 wt.%, even more preferably at least 35 wt.%, even more preferably at least 40 wt.%, even more preferably at least 45 wt.%, 50 wt.% of recycled content, preferably at least 60 wt.%, more preferably at least 70 wt.%, even more preferably at least 80 wt.%, most preferably at least 90 wt.% of recycled content based on the total weight of the absorbent article (100) excluding the core absorbent (30) material.

10. A package according to any of the preceding Claims, wherein the plastic film and/or layer composite has a thickness in the range from 10 $\mu$m to 300 $\mu$m, in particular in the range from 15 $\mu$m to 200 $\mu$m, preferably in the range from 20 $\mu$m to 180 $\mu$m, more preferably in the range from 25 $\mu$m to 175 $\mu$m, most preferably in the range from 30 $\mu$m to 60 $\mu$m as measured according to ISO 4593:1993.

11. A package according to any of the preceding Claims, wherein the plastic film and/or layer composite comprises at least substantially only one single plastic grade according to DIN EN ISO 11469, said plastic grade having a grade purity of at least 90 wt.%.

12. A package according to any of the preceding Claims, wherein the absorbent core (30) material comprises superabsorbent polymers with a bio-based content from about 5% to about 100% using ASTM D6866-10, method B.

13. A package according to any of the preceding Claims wherein at least one of the topsheet (26) or the backsheet (28) and at least one of the backsheet film, the backsheet nonwoven, the landing zone (44), the stretch ear film, the back ear nonwoven, the tape tabs (46), the ear hooks, the front ear nonwoven (47), the waist feature nonwoven, the waist feature elastics, the barrier leg cuff nonwoven (32), the outer leg elastics (34), the acquisition material (38), the core wrap nonwoven, the chassis adhesives, the core adhesives, or the elastic waist panels comprise a bio-based polyolefin with: a bio-based content from 5% to 100% using ASTM D6866-10, method B; and a purity of about 98 weight % polyolefin and less than 2 weight % impurities

14. A package according to any of the preceding Claims, wherein the absorbent articles (100) comprise a polyolefin selected from polyethylene and polypropylene, preferably polypropylene, and a polyolefin content of at least 90% by weight, based on a total weight of the absorbent article (100) excluding the absorbent core (30)material; and wherein recycled polyolefin recovered from an entirety of the absorbent article via a recycling operation comprises a polyolefin content of at least about 90 wt.%, based on a total weight of the absorbent article (100) excluding the absorbent core (30) material.

FIG. 1

FIG. 2

Europäisches
Patentamt

European
Patent Office

Office européen
des brevets

**EUROPEAN SEARCH REPORT**

Application Number

EP 24 20 2533

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 603 920 A1 (UNICHARM CORP [JP]) 5 February 2020 (2020-02-05) | 2,4-12, 14 | INV. B32B27/08 |
| Y | * paragraphs [0005], [0034], [0051], [0052], [0060], [0062], [0085], [0090], [0097], [0101], [0112]; figure 9 * | 1 | A61F13/49 A61F13/496 A61F13/551 B32B27/32 |
| | ----- | | |
| Y | US 2023/166488 A1 (KOHLWEYER CHRISTIAN [DE] ET AL) 1 June 2023 (2023-06-01) * paragraphs [0032] - [0036], [0101], [0107] * | 1 | |
| | ----- | | |
| A | WO 2023/195484 A1 (ASAHI CHEMICAL IND [JP]) 12 October 2023 (2023-10-12) * paragraphs [0001], [0013], [0062] * & EP 4 506 401 A1 (ASAHI CHEMICAL IND [JP]) 12 February 2025 (2025-02-12) | 1 | |
| | ----- | | |

**TECHNICAL FIELDS SEARCHED (IPC)**

B32B
B65F
A61F

The present search report has been drawn up for all claims

| Place of search | Date of completion of the search | Examiner |
|---|---|---|
| The Hague | 12 March 2025 | Aspeby, Erika |

EPO FORM 1503 03.82 (P04C01)

## ANNEX TO THE EUROPEAN SEARCH REPORT
## ON EUROPEAN PATENT APPLICATION NO.

EP 24 20 2533

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2025

| Patent document cited in search report | | Publication date | Patent family member(s) | | Publication date |
|---|---|---|---|---|---|
| EP 3603920 | A1 | 05-02-2020 | CN | 110337350 A | 15-10-2019 |
| | | | EP | 3603920 A1 | 05-02-2020 |
| | | | EP | 3862164 A1 | 11-08-2021 |
| | | | JP | 6865619 B2 | 28-04-2021 |
| | | | JP | 2018171780 A | 08-11-2018 |
| | | | US | 2020001506 A1 | 02-01-2020 |
| | | | WO | 2018179618 A1 | 04-10-2018 |
| US 2023166488 | A1 | 01-06-2023 | CN | 118284653 A | 02-07-2024 |
| | | | EP | 4441125 A1 | 09-10-2024 |
| | | | JP | 2024540538 A | 31-10-2024 |
| | | | US | 2023166488 A1 | 01-06-2023 |
| | | | WO | 2023102371 A1 | 08-06-2023 |
| WO 2023195484 | A1 | 12-10-2023 | CN | 119031896 A | 26-11-2024 |
| | | | EP | 4506401 A1 | 12-02-2025 |
| | | | JP | WO2023195484 A1 | 12-10-2023 |
| | | | WO | 2023195484 A1 | 12-10-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070219521
- US 20110139658
- US 20110139657
- US 20110152812
- US 20110139662
- US 20110139659
- US 20140005020
- US 9421137 B
- US 20130211363
- US 20170203542
- US 8703450 B
- US 9630901 B
- US 9822197 B